(19) Europäisches Patentamt
European Patent Office
Office européen des brevets

(11) Publication number: 0 105 885
B1

(12) **EUROPEAN PATENT SPECIFICATION**

(45) Date of publication of patent specification: 25.03.87

(51) Int. Cl.$^4$: **C 07 C 69/14,** C 07 C 67/08

(21) Application number: 83901169.9

(22) Date of filing: 16.03.83

(86) International application number:
PCT/US83/00361

(87) International publication number:
WO 83/03825 10.11.83 Gazette 83/26

# (54) PROCESS FOR THE PREPARATION OF METHYL ACETATE.

(30) Priority: 26.04.82 US 371626

(43) Date of publication of application:
25.04.84 Bulletin 84/17

(45) Publication of the grant of the patent:
25.03.87 Bulletin 87/13

(84) Designated Contracting States:
BE DE FR GB NL

(58) References cited:
US-A-1 939 116

(73) Proprietor: EASTMAN KODAK COMPANY
343 State Street
Rochester New York 14650 (US)

(72) Inventor: AGREDA, Victor Hugo
4239 Skyland Lane
Kingsport, TN 37664 (US)
Inventor: PARTIN, Lee Reynolds
1037 Watauga Street
Kingsport, TN 37660 (US)

(74) Representative: Baron, Paul Alexander Clifford
et al
Kodak Limited Patent Department Headstone
Drive
Harrow Middlesex HA1 4TY (GB)

EP 0 105 885 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to the manufacture of methyl acetate by a process which can be referred to as "reactive distillation". In this process glacial acetic acid reacts with methanol in a reaction zone and the product of the reaction, methyl acetate, is removed from the reaction zone by distillation.

U.S. Patent No. 1,400,849 discloses the reaction of dilute acetic acid with methanol in a reaction-distillation column. The dilute acetic acid and methanol are fed at opposite ends of the column and continuously flow in countercurrent fashion. In this type of process, the formation of azeotropes such as methyl acetate/water and methyl acetate/methanol azeotropes cannot be avoided. The result is a highly impure methyl acetate product. The process disclosed in U.S. Patent 1,400,849 employs a dephlegmator (condenser) to purify the distillate product from the reaction zone. While the dephlegmator may be effective to remove small amounts of methanol, it is incapable of "breaking" the aforementioned azeotropes. U.S. Patent 1,400,849 also discloses reacting glacial acetic acid with methanol in a distillation column which includes a single inlet pipe leading from a mixing tank in which the reactants are premixed. Such a system likewise yields an impure methyl acetate product and results in low conversion of the reactants.

It is an objective of this invention to provide a reactive distillation process of the general type disclosed in U.S. Patent 1,400,849 which produces highly pure methyl acetate with high reactant conversions. This process comprises a) continuously reacting glacial acetic acid with at least a stoichiometric amount of methanol in a reaction zone using an acidic catalyst, b) continuously removing a methyl acetate stream from the zone by distillation, and is characterized in that the glacial acetic acid and methanol are countercurrently flowed through the zone at rates such that the residence time is at least two hours. The residence time of the reacting liquids is selected to allow the glacial acetic acid to function as an extractive agent within the zone for water and/or methanol.

The details of this invention will be described hereinafter with respect to the accompanying drawings, in which

Figure 1 is a schematic flow diagram illustrating the invention and indicating apparatus for use therein.

Figure 2 is a schematic diagram of a reaction zone, specifically a reaction-distillation column, useful in this invention and showing its functional sections, i.e. a methanol/water stripping section, a reactive distillation section, an extractive distillation section and a methyl acetate/acetic acid rectification section.

Figure 3 is an illustration of a bubble cap tray design which may be used in the invention.

The esterification reaction of this invention, i.e. the reaction of glacial acetic acid with methanol in the presence of an acidic catalyst, which produces methyl acetate and water is well-known. It can be represented by the following:

$$NeOH + HOAc \xrightarrow{H+} MeOAc + H_2O$$

In the process of the invention, methanol (MeOH) and acetic acid (HOAc) are reacted in a reaction zone such as a continuous reaction-distillation column to achieve high conversion of the reactants to high purity methyl acetate product. For convenience, the invention will be described herein with reference to a reaction-distillation column. The aforementioned conversion is at least 99 percent, for example a conversion of acetic acid of 99.8 percent and a conversion of methanol of about 99.5 percent. Likewise, the purity of the product stream is such that it contains at least 99 percent methyl acetate, preferably, at least 99.5 percent methyl acetate. Typically, the product stream from the reaction-distillation column contains approximately 99.5 percent methyl acetate, 0.33 percent water, 0.15 percent methyl alcohol and 0.02 percent methyl propionate. Unless otherwise specified, all percentages used herein are weight percentages.

The high conversions of methyl alcohol and acetic acid to high purity methyl acetate are promoted by the countercurrent flow of reactants and products within the reaction-distillation column. Glacial acetic acid (i.e., acetic acid containing less than about 0.5 percent water) is fed to the upper part of the column, and methanol is fed to the lower end of the column. The reactants flow counter-currently through the column, reacting and flashing at each stage. The removal of methyl acetate, by flashing in preference to other components, at each reactive-distillation stage increases the extent of reaction achieved in each such stage.

As methyl acetate is formed from the reactants, the formation of azeotropes also occurs. For example, there is formed an azeotrope of methyl acetate and water which contains about 5 percent water, and an azeotrope of methanol and methyl acetate which contains about 19 percent methanol. In the practice of this invention, the glacial acetic acid functions as an extractive agent for water and methanol in the reaction zone to remove water and methanol from such azeotropes which improves the purity of the product stream. The removal of methanol from the methyl acetate/methanol azeotrope is also, and primarily, accomplished by reaction of the methanol with the acetic acid.

The residence time of the reacting liquids in the reaction zone is selected to allow or permit the glacial acetic acid to perform its extractive function. This residence time is the average duration of the reaction between reacting liquids in the reaction zone. It is conveniently expressed in hours and can be determined by dividing the volume of reacting liquids (e.g. the clear liquid volume in reaction trays where such trays are used) by the sum of the volumetric flow rates of

the glacial acetic acid and the methanol. The minimum residence time used in the process of this invention is subject to variation depending upon such things as the nature of the catalyst, catalyst concentration and the number of trays or stages within the reaction zone. For example, at a catalyst feed rate of 1 kg. of sulfuric acid per 100 kg. of glacial acetic acid, the residence time is typically at least 2 hours. Preferably, the residence time is approximately 2.4 hours. One convenient method for attaining the deisred residence time in the reaction zone is to employ reverse flow trays having high weirs, high bubble cap risers and large inlet and flow reversing zone sumps. A preferred design for such trays is shown in Figure 3, which will be discussed in detail hereinafter.

Intimate contact occurs in the reaction-distillation column between the acetic acid and methanol, between the acetic acid and methyl acetate/water azeotrope, and between the acetic acid and sulfuric acid and the methyl acetate/methanol azeotrope. Such intimate contact between acetic acid and each of these components promotes high conversions of reactants and high product purity. Accordingly, a high purity methyl acetate stream is continuously removed from the top of the column while by-product water is continuously removed from the bottom.

The acetic acid and methanol reactants are normally provided to the reaction-distillation column in approximately stoichiometric quantities. Typically, the acetic acid and methanol are fed in a molar ratio of about 1:1. However, a stoichiometric excess of methanol can be used (e.g., a molar ratio of methanol:acetic acid in the range of 1.1—2.0). Excess methanol is easily removed from the bottom of the column with the by-product water and can be recycled to the reaction zone following purification.

The methanol feed stream need not be pure methanol. For example, it may include water, as an impurity. The introduction of such water into the column does not adversely affect the operation of the process. Likewise, the glacial acetic acid feed stream may contain small amounts of impurities such as ethyl acetate, propionic acid and n-propyl acetate.

The most effective and economical catalyst for the process of this invention is sulfuric acid. Preferably, the catalyst is fed to the column as 95—98 percent sulfuric acid in aqueous solution. Other catalysts which have been used successfully include phosphoric acid and Amberlite 200™ acidic cation exchange resin. Catalysts such as phosphoric acid generally require the use of higher catalyst concentrations and/or larger size reactors. Also, the use of a cation exchange resin requiring packed sections in the column involves rather complex reactor designs.

The catalyst concentration introduced into the reaction-distillation column can be varied to provide the desired catalytic effect. When sulfuric acid is used as the catalyst, the flow rate found to be most beneficial in the process of this invention is about 1 kg. of sulfuric acid per 100 kg. of acetic acid feed stream. When higher catalyst concentrations are employed, shorter reaction times are required for a given conversion. However, higher catalyst concentrations give rise to increased corrosion rates. Conversely, lower catalyst concentrations give rise to lower corrosion rates but require longer reaction times for a given conversion.

The catalyst, e.g., sulfuric acid can be fed to the reaction-distillation column together with the acetic acid feed stream, or it may be fed by itself at a different location in the column. For example, it can be fed at the lower end of the extractive distillation section of the column. The location of the catalyst feed stream appears to affect the amount of water and/or methanol present in the product. Accordingly, the optimal placement of the catalyst feed stream in the column may vary depending upon the specific results desired.

Heat can be supplied to the reaction-distillation column by conventional means. Preferred methods include sparging steam into a base heater or the use of a reboiler. The use of a reboiler may require more exotic construction materials such as zirconium in order to minimize corrosion at the base heater. Highest corrosion is encountered in the lower half of the methanol stripping section of the column and in the base heater. High corrosion rates are due to the presence of aqueous sulfuric acid and the high temperatures normally found in the base of the column. To reduce such temperatures (and therefore the high corrosion rates encountered at the bottom of the column) the process can be operated by feeding an excess of methanol to the column, as discussed hereinbefore. The excess methanol can be separated from the by-product water and catalyst in a separate and smaller column operated at a lower pressure.

The reaction-distillation column is commonly operated at a temperature in the reaction section of 65° to 85°C. However, the optimal temperature for a particular process depends upon a number of factors, including the number of stages in the column, the desired production rate, the operating pressure and the corrosion rates which can be tolerated. Typically, it is desirable to maintain as low a temperature as possible for a given production rate. However, for a given production rate, less catalyst is required at a higher temperature. Upon a careful consideration of all of the variables in a given process, an optimal operating temperature will be apparent to one of ordinary skill in the art.

The reaction-distillation column is commonly operated at a pressure in the reaction section of 100 to 200 kPa (approximately 1 to 2 atmospheres). The optimal pressure for a particular process depends upon a number of factors, but operation of the process is normally easier and product quality is better at lower pressures.

In carrying out the process of this invention, the reflux ratio is normally at least 0.75. Distillation is most effective above this minimum reflux ratio. This reflux ratio, as known in the art, is the ratio of

overhead reflux flow rate to overhead product flow rate for the column. Preferred reflux ratios are 0.8 to 3.0 and most preferably 1.5 to 1.7. Conversion decreases rapidly at reflux ratios above 2.0.

Compounds that boil between the boiling points of glacial acetic acid and methanol, i.e. "intermediate boiling compounds" that enter the reaction-distillation column as impurities in feedstocks or that are formed as products of reactions of impurities with reactants can accumulate in the column. They often accumulate in the upper portion of the reaction section and throughout the extractive distillation section of this column. Typical intermediate boiling compounds of this type are methyl propionate, methyl butyrate and isopropyl acetate. Such compounds exhibit boiling points which are considerably higher than the boiling point of methyl acetate. However, either the compounds themselves or their water azeotropes boil at considerably lower temperatures than water. In addition, the activity coefficients in water of the compounds or their azeotropes may be quite high (e.g., as in the case of methyl propionate). Therefore, such compounds or their azeotropes tend to build up in the column. The accumulation of these intermediate boiling compounds reduces the volume in the column available for reaction and also reduces the extractive capability of acetic acid. This causes lower conversions and produces a product having a higher concentration of water.

To overcome this difficulty, a vapor stream containing the intermediate boiling compounds is removed from the column and such intermediate boiling compounds are removed from the stream which is then returned to the column. The vapor stream is preferably withdrawn from the middle to upper part of the reaction section of the column as shown in Fig. 1. The vapor which is removed from the column is mostly methyl acetate and acetic acid but also contains some intermediate boiling compounds. The vapor also contains water and methanol, which are present predominantly as azeotropes with methyl acetate. The vapor is passed through wire mesh to remove entrained sulfuric acid, which is returned to the column. The vapor is then fed to a first column where methyl acetate and its azeotropes and the intermediate boiling compounds are taken overhead, while acetic acid and water are collected as bottoms from the column and are returned to the reaction-distillation column. The distillate from the first column is then fed to a second column where the intermediate boiling compounds are obtained as the underflow. Methyl acetate and its azeotropes are taken as the distillate of this column and are returned to the reaction-distillation column.

A holding zone such as a tank in which the reaction liquid is temporarily held can be used in the process of this invention to obtain some increase in reactant conversions. Thus, the reactant conversion, for a given number of trays, can be increased by feeding at least a portion of the reaction mixture from the reaction-distillation column to a tank having a large reaction liquid hold-up (residence time). The tank is preferably located between the reaction section and the methanol stripping section of the reaction-distillation column. However, the location and size of the tank are not very significant features of this invention and the elimination of such a tank decreases the acetic acid conversion only slightly. When such a tank is employed in the process of this invention, it usually provides an additional residence time of about one hour.

In practicing the invention, the choice of methanol feed location depends on the water content of the methanol and on whether the methanol is fed as a liquid or vapor. The optimal point of methanol feed will be apparent to one of ordinary skill in the art.

It is difficult to achieve precise control of the reaction-distillation column overhead stream and bottom composition simply by measuring the flow rate of the acetic acid and methanol feed streams and of the distillate stream. These flow rates must be adjusted frequently due to such things as changes in feed stream and product compositions and liquid density changes due to variations in temperature. However, it has been found that the overhead and bottom compositions can be controlled quite easily using two temperature control loops.

The first and most important loop controls the methyl acetate distillate flow rate and typically varies it within ±5 percent of a "target distillate flow rate". The target distillate flow rate is dependent upon the reactant flow rates. Upon determining the required reactant flow rates for the desired production level (i.e., the target distillate flow rate), the distillate flow rate is controlled so that the temperature of a point in the middle to upper part of the reaction zone is maintained at a preselected constant level. The point should be located at a position where the reaction mixture composition, particularly the water concentration, changes sharply. This control scheme assures that the reaction zone is positioned within the reaction trays (which are designed to slow the progress of the liquid reaction medium within the reaction zone and provide a high liquid hold-up time), thus ensuring high conversion and proper extraction of water and methanol. The exact temperature of this control point is dictated by the concentration at which the intermediate boiling impurities are allowed to accumulate in the reaction-distillation column. The desired point can be easily determined by one skilled in the art.

A similar method is employed to control the methanol feed stream. A second temperature feedback loop is used to vary the methanol feed stream within about ±10 percent of the "target methanol feed rate" for a given methyl acetate production rate during stoichiometric reaction. This second temperature control point is located in the lower section of the methanol stripping section of the reaction-distillation column, where the temperature changes rapidly with slight

changes in methanol concentration. As stated previously, the methyl acetate production rate is determined by the reactant feed rates. Therefore, for a given target methyl acetate production rate, the methanol feed rate should equal the stoichiometric requirements plus the anticipated methanol loss due to the presence of methanol in the by-product water stream and the methyl acetate product stream. This is the aforementioned target methanol flow rate.

It is important that the distillate and methanol flows not be varied significantly from the indicated ranges for a given production rate. The high residence time of reacting liquid in the reaction-distillation column makes the response of such column very sluggish. Therefore, if the distillate and/or the methanol flow rates are allowed to vary over a wide range, it is very difficult to maintain the desired production rate. When excess methanol is used in the process, the control on the second loop can be eased.

The temperature controls discussed previously operate to control the reaction within the reaction-distillation column. As a result, only minor amounts of water and methanol get into the product streams, and mere traces of acetic acid (typically less than 0.3 percent) and methanol (typically less than 0.1 percent) are detected in the by-product water stream when no excess methanol is fed to the reaction-distillation column.

The remaining operating parameters can be controlled in the same manner as they are in a typical distillation column. Turbine meters are recommended to measure the flows of acetic acid, methanol, and methyl acetate distillate because their accuracy allows the materials balance of the process to be checked to ensure proper control of the process. Orifice meters can be used to measure the reflux flow rate, the sulfuric acid flow rate, and the steam flow to the base of the column. A back-up meter is normally provided in case the sulfuric acid orifice meter fails. Maintenance of steady catalyst flow is necessary for smooth operation of the process.

A continuous on-line water analyzer, calibrated for 0—10,000 ppm water in methyl acetate, can be installed on the distillate line. This instrument should have a high water concentrate alarm. The information provided by such an analyzer is valuable for the smooth operation of the process and the assurance of high quality product.

Normally the reaction-distillation column has approximately 30 trays, often up to about 45 trays or more in the reaction section. These trays are preferably of the bubble cap type. Approximately 10 trays each are normally required for the acetic acid rectification section, the extractive distillation section, and the methanol stripping section.

The invention will now be described with reference to the drawings. Elements are labeled consistently where they appear in more than one drawing.

Figure 1 depicts a flow diagram of the process of this invention. The reaction-distillation column *1* is provided in its upper section with an inlet for glacial acetic acid which is provided to the column through feed stream *3*. Methanol is fed to the lower part of the reaction section of the column through feed pipe *5*. Sulfuric acid catalyst is fed through line *7* to the lower portion of the extractive distillation section of the column. Steam is applied to the base of the column through line *9*.

A vapor stream (sometimes referred to as a sidedraw stream) is withdrawn from the middle to upper part of the reaction section of the column through line *11*. The vapor stream is passed through wire mesh separator *12*, from which entrained sulfuric acid is returned to the column through line *13*, and the remaining components of the stream are fed through line *14* to a first distillation column *15*. A condensate stream comprising mostly acetic acid and water is taken from the bottom of column *15* and is returned through line *17* to reaction-distillation column *1*. Methyl acetate and its azeotropes and intermediate boiling compounds are fed through line *18* to a second distillation column *19*. Methyl acetate and its azeotropes are taken as the distillate from column *19* and are returned through line *21* to the reaction-distillation column *1*. The intermediate boiling compounds are removed from column *19* through line *23*.

The reaction mixture from the lower end of the reaction section of reaction-distillation column *1* is fed through line *24* to tank *25*. Tank *25* has a liquid hold-up time (residence time) of at least one hour and may be used to increase the reactant conversion for a given number of trays in reaction-distillation column *1*. The reaction mixture is returned from tank *25* to reaction-distillation column *1* through line *26*. The vapor product from tank *25* is returned to reaction-distillation column *1* through line *27*.

By-product water, sulfuric acid, and excess methanol, if any, are withdrawn from the base of reaction-distillation column *1* through line *28*. Methyl acetate product stream is withdrawn from the top of column *1* through line *29* and condensed in condenser *30*. A portion of the condensate (reflux stream) is returned to the column through line *31*, and a very pure methyl acetate product stream is taken off through line *32*.

The flow diagram represented by Figure 1 and described herein is a simplified flow diagram of the process of this invention. Apparatus used in the process may additionally include vent scrubbers and other well-known apparatus.

Figure 2 is a schematic diagram in detail of reaction-distillation column *1* showing the various functional sections within the column. These functional sections are methanol/water stripping section *33*, reactive-distillation section *35*, extractive distillation section *37*, and methyl acetate/acetic acid rectification section *39*.

The area labeled *33* represents the approximate limits of the methanol/water stripping section of the column. In this section, methanol is separated or stripped from the by-product water. The water is removed from the column through line *28* while

the methanol ascends through the column, reacting with acetic acid.

The region of the column designated *35* is the reactive distillation section of the column. In this area, much of the reaction between acetic acid and methanol occurs. The methyl acetate product is flash distilled at each stage and rises through the column. During this process, methyl acetate/water and methyl acetate/methanol azeotropes form. These azeotropes are "broken" (separated into their component parts) by the acetic acid which extracts water and methanol as it descends through the column and as it also reacts with the methanol.

The extractive distillation section of the column is indicated by number *37*. This region is rich in acetic acid and formation of methyl acetate continues to occur therein. The breaking of the aforementioned azeotropes also continues in this section.

In methyl acetate/acetic acid rectification section *39* the methyl acetate product is separated from acetic acid reactant. The acetic acid descends through the column while the methyl acetate product exits through line *29*.

In one embodiment of this process, for a given production rate, methanol stripping section *33* comprises 12 reverse flow-valve trays spaced 46 cm apart; reactive distillation section *35* comprises 60 reverse flow-bubble cap trays spaced 64 cm apart; extractive distillation section *37* comprises 10 crossflow-valve trays spaced 46 cm apart; and methyl acetate/acetic acid rectification section *39* comprises 13 crossflow-valve trays spaced 46 cm apart. Such an arrangement has been found to operate very efficiently.

A preferred reverse flow-bubble cap tray design for obtaining a hold-up in the flow of liquid reactant, i.e., regulating its flow, during the process of the invention is depicted in Figure 3. The spacing *41* between vertically adjacent trays is preferably 64 cm. Outlet weir *43* for the tray is preferably 13 cm in height. Outlet weir *45* for the tray is also preferably 13 cm in height. In order to obtain the desired flow and the maximum bubbling area, the tray includes a 38 cm high baffle *47* through the center of the tray. The tray design includes an inlet sump *48* which is preferably 25 cm deep. The flow of liquid from the inlet sump is designated by arrows *49*. The liquid flows up from inlet sump *48*, around baffle *44*, through bubbling area *50* on one side of baffle *47*, through another 25 cm sump *51* at the opposite end of the tray from inlet sump *48*, passing under a 25 cm baffle *53*, through bubbling area *50* on the other side of baffle *47*, and over outlet weir *45*, and then falls to the inlet sump of the tray below. Bubbling area *50*, on both sides of baffle *47*, contains a plurality of bubble caps *55*. While only a few bubble caps are illustrated in Figure 3, the entire bubbling area *50* may be covered with bubble caps, the exact number and positioning of which will be apparent to one of ordinary skill in the art. Preferably, bubble caps *55* measure 10 cm in diameter and have 13 cm risers. Each tray contains on the order of 150 to 200 bubble caps.

**Claims**

1. A process for preparing methyl acetate which comprises:

a) continuously reacting glacial acetic acid with at least a stoichiometric amount of methanol in a reaction zone using an acidic catalyst, and

b) continuously removing a methyl acetate stream from the zone by distillation,

characterized in that the glacial acetic acid and methanol are countercurrently flowed through the zone at rates such that the residence time is at least two hours.

2. The process according to Claim 1 in which the acidic catalyst is sulfuric acid, phosphoric acid or an acid cation exchange resin.

3. The process according to claim 1 or claim 2 in which the acidic catalyst is sulfuric acid which is introduced into the reaction zone at a rate of 1 kg per 100 kg of the acetic acid.

4. The process according to any of Claims 1—3 in which the methyl acetate stream is condensed and a portion of the condensate is returned to the reaction zone at a reflux ratio of at least 0.75.

5. The process according to any of Claims 1—4 in which a vapor stream containing intermediate boiling compounds, such as methyl propionate, methyl butyrate, isopropyl acetate or mixtures thereof is withdrawn from the reaction zone, intermediate boiling compounds are removed from the stream and the stream is returned to the reaction zone.

6. The process according to any of Claims 1—5 in which a portion of the reaction mixture is removed from the reaction zone, placed in a holding zone for at least 1 hour and then returned to the reaction zone.

**Patentansprüche**

1. Verfahren zur Herstellung von Methylacetat, bei dem man:

a) Eisessig kontinuierlich mit mindestens einer stöchiometrischen Menge Methanol in einer Reaktions zone unter Verwendung eines sauren Katalysators umsetzt und

b) kontinuierlich einen Methylacetatstrom aus der Zone durch Destillation abzieht,

dadurch gekennzeichnet, daß man den Eisessig und das Methanol im Gegenstrom mit Geschwindigkeiten durch die Zone fließen läßt, daß die Verweilzeit mindestens 2 Stunden beträgt.

2. Verfahren nach Anspruch 1, dadurch gekennzeichnet, daß man als Katalysator Schwefelsäure, Phosphorsäure oder ein saures Kationenaustauscherharz verwendet.

3. Verfahren nach Anspruch 1 oder 2, dadurch gekennzeichnet, daß man als sauren Katalysator Schwefelsäure verwendet, die in die Reaktionszone mit einer Geschwindigkeit von 1 kg pro 100 kg Essigsäure eingespeist wird.

4. Verfahren nach einem der Ansprüche 1—3,

dadurch gekennzeichnet, daß der Methylacetatstrom kondensiert und ein Teil des Kondensates in die Reaktionszone bei einem Rückflußverhältnis von mindestens 0,75 zurückgeführt wird.

5. Verfahren nach einem der Ansprüche 1—4, dadurch gekennzeichnet, daß ein Dampfstrom, der intermediär siedende Verbindungen wie Methylpropionat, Methylbutyrat, Isopropylacetat oder Mischungen hiervon enthält, aus der Reaktionszone abgezogen wird, daß intermediär siedende Verbindungen aus dem Strom entfernt werden und daß der Strom in die Reaktionszone zurückgeführt wird.

6. Verfahren nach einem der Ansprüche 1—5, dadurch gekennzeichnet, daß ein Teil der Reaktionsmischung aus der Reaktionszone abgezogen, mindestens eine Stunde lang in einer Verweilzone aufbewahrt und dann in die Reaktionszone zurückgeführt wird.

**Revendications**

1. Procédé de préparation d'acétate de méthyle consistant à:

a) faire réagir de façon continue de l'acide acétique glacial avec au moins la quantité stoechiométrique de méthanol, dans une zone de réaction, en utilisant un catalyseur acide, et

b) éliminer de façon continue de la zone de réaction par distillation, le flux d'acétate de méthyle,

caractérisé en ce que l'acide acétique glacial et le méthanol circulent à contre-courant dans la zone de réaction, à des vitesses telles que le temps de résidence est d'au moins deux heures.

2. Procédé conforme à la revendication 1, dans lequel le catalyseur acide est l'acide sulfurique, l'acide phosphorique ou une résine acide échangeuse de cation.

3. Procédé conforme aux revendications 1 ou 2, dans lequel le catalyseur acide est l'acide sulfurique, et il est introduit dans la zone de réaction à la vitesse de 1 kg pour 100 kg d'acide acétique.

4. Procédé conforme à l'une quelconque des revendications 1 à 3, dans lequel le flux d'acétate de méthyle est condensé, et une partie du condensat retourne dans la zone de réaction, dans un rapport de reflux d'au moins 0,75.

5. Procédé conforme à l'une quelconque des revendications 1 à 4, dans lequel le flux de vapeurs contenant les composés de points d'ébullition intermédiaires, tels que le propionate de méthyle, le butyrate de méthyle, l'acétate d'isopropyle, ou des mélanges de ces composés, est retiré de la zone de réaction, les composés de points d'ébullition intermédiaires sont éliminés du flux, puis ce dernier est renvoyé vers la zone de réaction.

6. Procédé conforme à l'une quelconque des revendications 1 à 5, dans lequel une partie du mélange réactionnel est retirée de la zone de réaction, placée dans une zone d'attente pendant au moins 1 heure, puis renvoyée vers la zone de réaction.

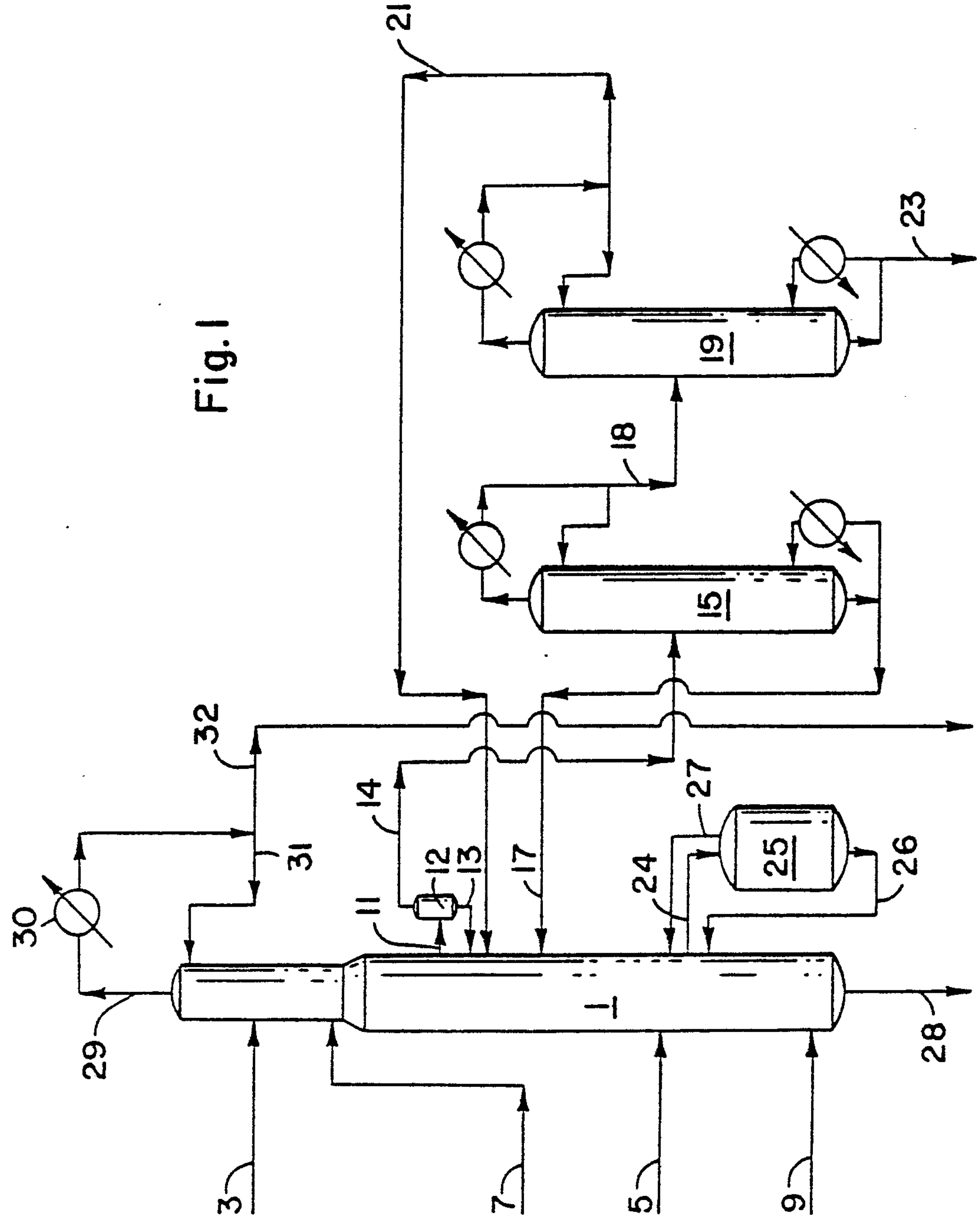


Fig. 1

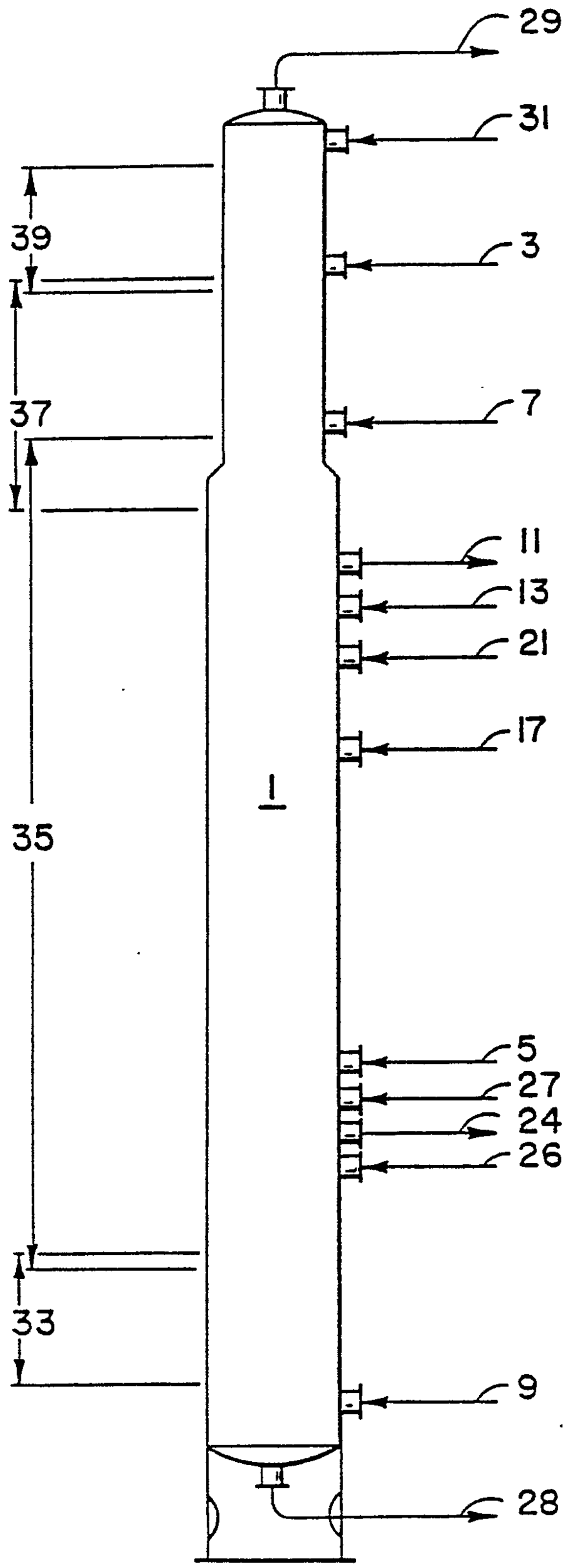


Fig. 2

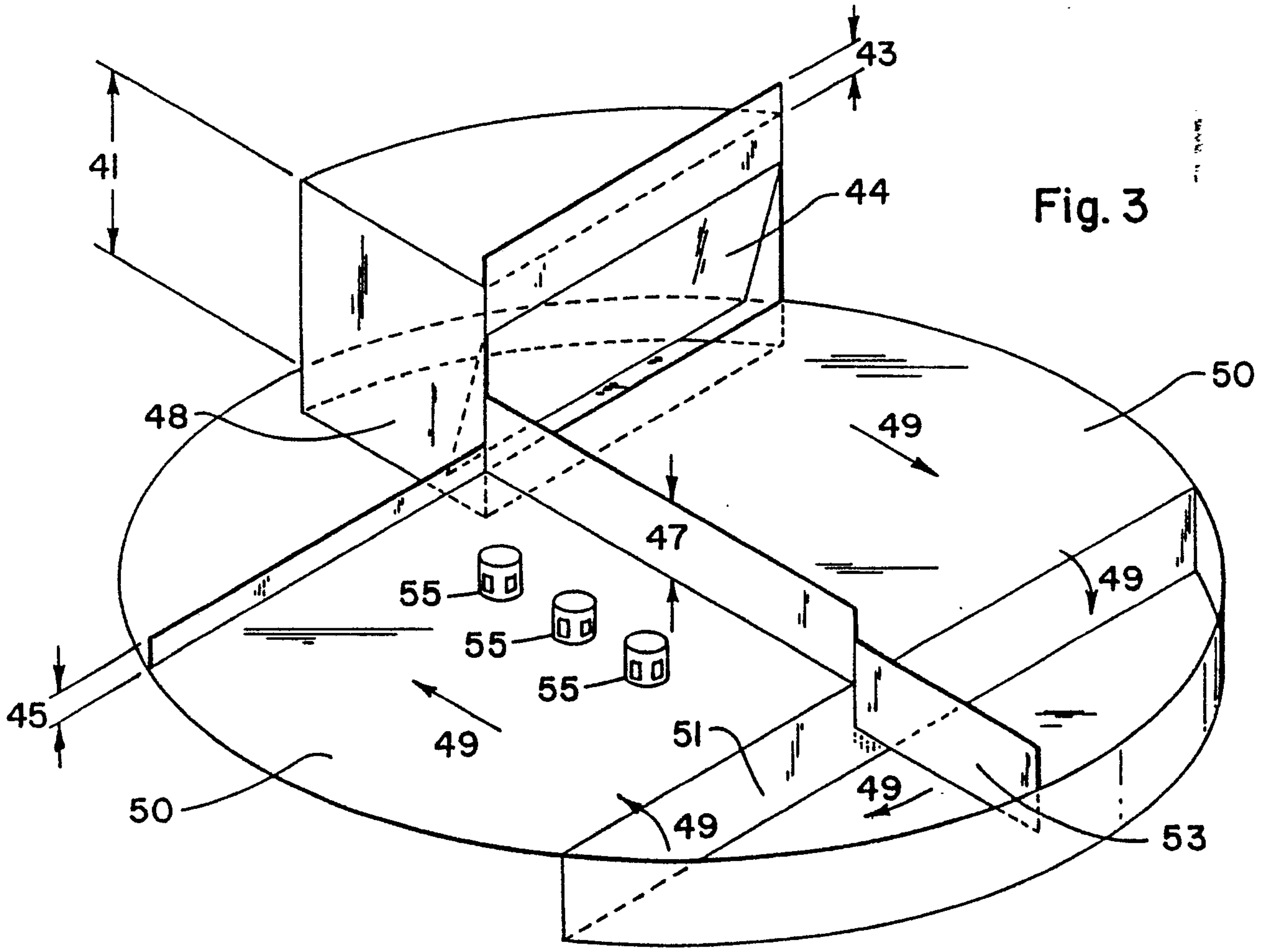


Fig. 3